# EUROPEAN PATENT SPECIFICATION

(11) **EP 0 785 927 B1**
(45) Date of publication and mention of the grant of the patent: **27.08.2003**
(21) Application number: 95940634.9
(22) Date of filing: 11.10.1995
(51) Int. Cl.: C07D 263/57

(54) **NOVEL BENZOXAZOLES**
NEUE BENZOXAZOLE
NOUVEAUX BENZOXAZOLES

(30) Priority: 12.10.1994 US 321730; 06.06.1995 US 467091
(43) Date of publication of application: 30.07.1997
(73) Proprietor: Euroceltique S.A., L-2330 Luxembourg (LU)
(72) Inventor: CAVALLA, David, J., Cambridge CB1 2DX (GB); CHASIN, Mark, Manalapan, NJ 07726 (US); HOFER, Peter, CH-4410 Liestal (CH); DOLBY, Lloyd, Eugene, OR 97405 (US)
(74) Representative: Ruffles, Graham Keith
(86) International application number: US9514399
(87) International publication number: WO96011917

(56) References cited:
- EP-A- 0 332 988
- DE-A- 2 008 414
- DE-A- 2 314 676
- DE-A- 2 346 034
- JP-A- 57 021 375
- US-A- 2 320 654
- US-A- 3 136 771
- US-A- 3 470 164
- US-A- 3 491 091
- US-A- 3 491 106
- US-A- 3 494 919
- US-A- 3 541 100
- US-A- 3 574 218
- US-A- 3 586 670
- US-A- 3 647 812
- US-A- 3 666 769
- US-A- 3 669 979
- US-A- 3 674 781
- US-A- 3 706 834
- US-A- 4 020 165
- US-A- 4 025 636
- US-A- 4 025 637
- US-A- 4 167 628
- US-A- 4 416 892
- US-A- 4 652 654
- US-A- 4 732 978
- US-A- 4 831 152
- US-A- 4 910 211
- US-A- 5 190 942
- US-A- 5 206 255
- US-A- 5 322 847
- CHEM. PHARM. BULLETIN, Volume 31, No. 9, issued 1983, ISOMURA et al., "Studies on the Synthesis and Antiinflammatory Activity of 2, 6 D. Tert Butylphenols with a Heterocyclic Group at the 4 Position, I", pages 3168-3178.

## Description

### BACKGROUND OF THE INVENTION

Asthma is a complex disease involving the concerted actions of multiple inflammatory and immune cells, spasmogens, inflammatory mediators, cytokines and growth factors. In recent practice there have been four major classes of compounds used in the treatment of asthma, namely bronchodilators (e.g., β-adrenoceptor agonists), anti-inflammatory agents (e.g., corticosteroids), prophylactic anti-allergic agents (e.g., cromolyn sodium) and xanthines (e.g., theophylline) which appear to possess both bronchodilating and anti-inflammatory activity.

Theophylline has been a preferred drug of first choice in the treatment of asthma. Although it has been touted for its direct bronchodilatory action, theophylline's therapeutic value is now believed to also stem from anti-inflammatory activity. Its mechanism of action remains unclear. However, it is believed that several of its cellular activities are important in its activity as an anti-asthmatic, including cyclic nucleotide phosphodiesterase inhibition, adenosine receptor antagonism, stimulation of catecholamine release, and its ability to increase the number and activity of suppressor T-lymphocytes. While all of these actually may contribute to its activity, only PDE inhibition may account for both the anti-inflammatory and bronchodilatory components. However, theophylline is known to have a narrow therapeutic index, and a wide range of untoward side effects which are considered problematic.

Cyclic nucleotide phosphodiesterases (PDEs) have received considerable attention as molecular targets for anti-asthmatic agents. Cyclic 3',5'-adenosine monophosphate (cAMP) and cyclic 3',5'-guanosine monophosphate (cGMP) are known second messengers that mediate the functional responses of cells to a multitude of hormoves, neurotransmitters and autocoids. At least two therapeutically important effects could result from phosphodiesterase inhibition, and the consequent rise in intracellular adenosine 3',5'-monophosphate (cAMP) or guanosine 3',5'-monophosphate (cGMP) in key cells in the pathophysiology of asthma. These are smooth muscle relaxation (resulting in bronchodilation) and anti-inflammatory activity.

It has become known that there are multiple, distinct PDE isoenzymes which differ in their cellular distribution. A variety of inhibitors possessing a marked degree of selectivity for one isoenzyme or the other have been synthesized.

The structure-activity relationships (SAR) of isozyme-selective inhibitors has been discussed in detail, e.g., in the article of Theodore J. Torphy, et al., "Novel Phosphodiesterase Inhibitors For The Therapy Of Asthma", Drug News & Perspectives, 6(4) May 1993, pages 203-214. The PDE enzymes can be grouped into five families according to their specificity toward hydrolysis of cAMP or cGMP, their sensitivity to regulation by calcium, calmodulin or cGMP, and their selective inhibition by various compounds. PDE I is stimulated by Ca²⁺/calmodulin. PDE II is cGMP-stimulated, and is found in the heart and adrenals. PDE III is cGMP-inhibited, and inhibition of this enzyme creates positive inotropic activity. PDE IV is cAMP specific, and its inhibition causes airway relaxation, anti-inflammatory and antidepressant activity. PDE V appears to be important in regulating cGMP content in vascular smooth muscle, and therefore PDE V inhibitors may have cardiovascular activity.

While there are compounds derived from numerous structure activity relationship studies which provide PDE III inhibition, the number of structural classes of PDE IV inhibitors is relatively limited. Analogues of rolipram, which has the following structural formula: 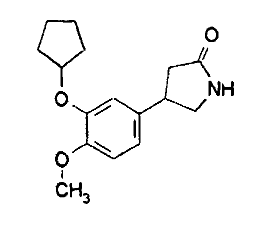 and of Ro-20-1724, which has the following structural formula: 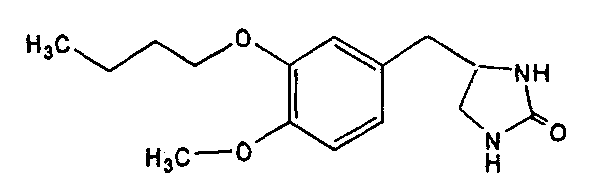 have been studied.

Rolipram, which was initially studied because of its activity as an antidepressant has been shown to selectively inhibit the PDE IV enzyme and this compound has since become a standard agent in the classification of PDE enzyme subtypes. There appears to be considerable therapeutic potential for PDE IV inhibitors. Besides initial work suggesting an anti-depressive action, rolipram has been investigated for its anti-inflammatory effects, particularly in asthma. In-vitro, rolipram, Ro-20-1724 and other PDE IV inhibitors have been shown to inhibit (1) mediator synthesis/release in mast cells, basophils, monocytes and eosinophils; (2) respiratory burst, chemotaxis and degranulation in neutrophils and eosinophils; and (3) mitogen-dependent growth and differentiation in lymphocytes (The PDE IV Family Of Calcium-Phosphodiesterases Enzymes, John A. Lowe, III, et al., Drugs of the Future 1992, 17(9):799-807).

PDE IV is present in all the major inflammatory cells in asthma including eosinophils, neutrophils, T-lymphocytes, macrophages and endothelial cells. Its inhibition causes down regulation of cellular activation and relaxes smooth muscle cells in the trachea and bronchus. On the other hand, inhibition of PDE III, which is present in myocardium, causes an increase in both the force and rate of cardiac contractility. These are undesirable side effects for an anti-inflammatory agent. Theophylline, a non-selective PDE inhibitor, inhibits both PDE III and PDE IV, resulting in both desirable anti-asthmatic effects and undesirable cardiovascular stimulation. With this well-known distinction between PDE isozymes, the opportunity for concomitant anti-inflammation and bronchodilation without many of the side effects associated with theophylline therapy is apparent. The increased incidence of morbidity and mortality due to asthma in many Western countries over the last decade has focused the clinical emphasis on the inflammatory nature of this disease and the benefit of inhaled steroids. Development of an agent that possesses both bronchodilatory and anti-inflammatory properties would be most advantageous.

It appears that selective PDE IV inhibitors should be more effective with fewer side effects than theophylline. Clinical support has been shown for this hypothesis.

Attempts have therefore been made to find new compounds having more selective and improved PDE IV inhibition.

### OBJECTS AND SUMMARY OF THE INVENTION

It is accordingly a primary object of the present invention to provide new compounds which are effective PDE IV inhibitors.

It is another object of the present invention to provide new compounds which act as effective PDE IV inhibitors with lower PDE III inhibition.

It is a further object of the present invention to provide new compounds which have a superior PDE IV inhibitory effect as compared to rolipram or other known compounds.

It is a further object of the present invention to provide new compounds which have a substantially equal or superior PDE IV inhibitory effect as compared to known chemical compounds, and which exhibit surprisingly greater selectivity with regard to their inhibitory effects.

It is another object of the present invention to provide a method of treating a patient requiring PDE IV inhibition.

It is another object of the present invention to provide new compounds for treating disease states associated with abnormally high physiological levels of cytokines, including tumor necrosis factor.

It is another object of the present invention to provide a method of synthesizing the new compounds of this invention.

It is another object of the present invention to provide a method for treating a mammal suffering from a disease state selected from the group consisting of asthma, allergies, inflammation, depression, dementia, a disease caused by Human Immunodeficiency Virus and disease states associated with abnormally high physiological levels of cytokines.

With the above and other objects in view, the present invention mainly comprises compounds of the formula: 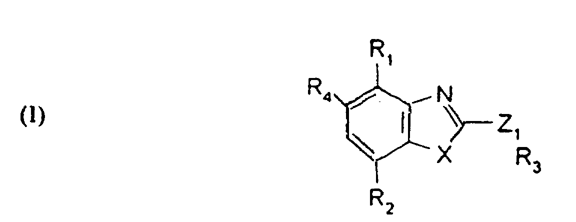 wherein
X is O or S;
R₁ and R₂ are independently selected from hydrogen, halogen, hydroxy, nitro, QZ₂, OQZ₂, OCOQZ₂, NHQZ₂ or NHCOQZ₂ wherein: Q is a bond, a straight-chain or branched alkylene, alkenylene or alkynylene group containing from 1 to 12 carbon atoms;
Z₂ is hydrogen, CH(OH)QH, OQH, NO₂, N(QH)₂, CO₂QH, CON(QH)₂, CON(OH)QH, OCOQH, OCON(QH)₂, OCON(OH)QH, NHCON(QH)₂, N(OH)CON(QH)₂, CH=NOCOQH, CH=NOCON(QH)₂, COQH, N(OH)COQH, or a 2-pyridyl being unsubstituted or further substituted with one or more halogen atoms, OH, OQH, NO₂, NH, CO₂QH, CON(QH)₂, OCOQH, and CON(QH)₂;
R₃ is an unsubstituted phenyl or phenyl substituted with 1-3 members independently chosen from the group consisting of OH, halogen, NH₂, NO₂, R₅ or R₆;
Z₁ is a linkage selected from a bond, -CH₂-, -CH=CH-, -CH₂CH₂-, -CH(CH₃)-and -C(CH₃)₂-; except that Z₁R₃ is not 3,5-di-t-butyl-4-hydroxy-phenyl;
R₄ is hydrogen or a halogen;
R₅ is an alkyl; and
R₆ is a cycloalkyl group of 1 to 12 carbon atoms;
provided that one or both of R₁ and R₂ are QZ₂ wherein Q is an alkenylene or alkynylene and Z₂ is a 2-pyridine.

The term "lower alkyl" is defined for purposes of the present invention as straight or branched chain radicals having from 1 to 3 carbon atoms.

In one preferred aspect of the invention, the compositions of Formula (I) are benzoxazole-based compounds having a PDE IV IC₅₀ of less than about 10µM.

### DETAILED DESCRIPTION

The compounds of the present invention, as demonstrated in the appended examples, are effective in the mediation or inhibition of PDE IV in humans and other mammals. Further, these compounds are selective PDE IV inhibitors which possess both bronchodilatory and anti-inflammatory properties substantially without undesirable cardiovascular stimulation caused by PDE III inhibition. Many of these compounds have a substantially equal or superior PDE IV inhibitory effect as compared to theophylline.

The present invention is further related to a method for the treatment of allergic and inflammatory disease which comprises administering to a mammal in need thereof an effective amount of the compounds of the present invention.

The present invention is also related to a method for the mediation or inhibition of the enzymatic or catalytic activity of PDE IV activity in mammals, particularly humans, which comprises administering an effective amount of the above-described compounds of the invention to a mammal in need of PDE IV inhibition.

The compounds of the present invention may find use in the treatment of other disease states in humans and other mammals, such as in the treatment of disease states associated with a physiologically detrimental excess of tumor necrosis factor (TNF). TNF activates monocytes, macrophages and T-lymphocytes. This activation has been implicated in the progression of Human Immunodeficiency Virus (HIV) infection and other disease states related to the production of TNF and other cytokines modulated by TNF. Accordingly, the compositions of the present invention can be administered in effective amounts to mammals suffering from asthma, allergies, inflammation, depression, dementia, atopic diseases, rhinitis and disease states associated with abnormally high physiological levels of cytokines, inflammatory cytokines and chemokines.

In certain preferred embodiments, R, is a halogen, such as chlorine; one of R₁ or R₂ is hydrogen and X is oxygen or sulfur and preferably oxygen.

In further preferred embodiments, Z₁ is a linkage selected from the group consisting of a bond, -CH₂-, -CH₂CH₂- and -CH=CH-.

In those aspects of the invention where one or both of R₁ and R₂ are QZ₂, Q is preferably an alkenylene or alkynylene group. Suitable alkenylene groups include, for example,-CH=CH-, and -CH₂-CH=CH-; suitable alkynyl groups include -C≡C-, and -C≡C-CH₂-.

Still further aspects of the invention include Q as an alkylene group. A nonlimiting list of suitable groups include -CH₂CH₂-, -CH₂CH₂CH₂-.

Within this aspect of the invention, any further group Z₂, where included, is preferably a 2-pyridine or 2-thiazole group.

R₃ can include an unsubstituted or substituted phenyl group such as phenyl, chlorophenyls, fluorophenyls, bichloro and bifluorophenyls, chloro-fluorophenyls and the like. Other aspects of the invention include R₃ groups such as 3,5-di-t-butyl-4-hydroxyphenyl; 3,5-di-t-butyl-4-acetoxyphenyl; 3,4-dimethoxyphenyl and 3-cyclopentyloxy-4-methoxyphenyl.

In another aspect of the invention, when X is O or S, preferably O and Z₁ is - CH₂-, R₃ is 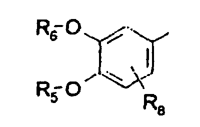 wherein
R₅ is hydrogen or branched or straight chain alkyl group of 1-12 carbon atoms, preferably lower alkyl, most preferably methyl or ethyl, and R₆ is a cycloalkyl of 3-6 carbon atoms, preferably cyclopentyl;
R₈ is hydrogen, lower alkyl or halogen.

In still further aspects of the inventions, R₃ includes moieties such as a chlorophenyl or a di-t-butyl-hydroxy benzyl.

A preferred compound of the present invention is:
5-chloro-2-(3,5-di-t-butyl-4-hydroxy-benzyl)-7-(2-(2-pyridyl)-ethynyl)-benzoxazole.

In other preferred aspects of the invention, the compounds in accordance with the present invention have a PDE IV IC₅₀ of less than about 10µM and more preferably less than that of rolipram.

Description of the synthesis of a representative number of these molecules is set forth in the Examples. The synthesis of other molecules not specifically shown in the examples but within the scope of the invention are carried out using those techniques shown with modifications which are known to those of ordinary skill in the art.

The compounds of the present invention have been found to be highly effective PDE IV inhibitors, the inhibition of which is in fact significantly and surprisingly greater than that of theophylline which exhibits 50% inhibition of PDE IV at around 350 µM. In addition, the concentration which yields 50% inhibition of PDE IV (IC₅₀) for the compound prepared in Example I is 0.6 µM, whereas the IC₅₀ for rolipram when run in the same assay was 2.8 µM. Historically, the IC₅₀ for rolipram is considered to be 3.5 µM. In any case, it is apparent that this inventive compound is several times as effective as a PDE IV inhibitor as compared to rolipram (or theophylline).

While the PDE III inhibition of an Example 1 compound is only 22% at 10 µM, it is clear that the compound of the invention is highly selective as a PDE IV inhibitor.

Accordingly, the compounds of the present invention can be administered to anyone requiring PDE IV inhibition. Administration may be orally, topically, by suppository, inhalation or insufflation, or parenterally.

The present invention also encompasses, where appropriate, all pharmaceutically acceptable salts of the foregoing compounds. One skilled in the art will recognize that acid addition salts of the presently claimed compounds may be prepared by reaction of the compounds with the appropriate acid via a variety of known methods. Alternatively, alkali and alkaline earth metal salts are prepared by reaction of the compounds of the invention with the appropriate base via a variety of known methods.

Various oral dosage forms can be used, including such solid forms as tablets, gelcaps, capsules, caplets, granules, lozenges and bulk powders and liquid forms such as emulsions, solution and suspensions. The compounds of the present invention can be administered alone or can be combined with various pharmaceutically acceptable carriers and excipients known to those skilled in the art, including but not limited to diluents, suspending agents, solubilizers, binders, disintegrants, preservatives, coloring agents, lubricants and the like.

When the compounds of the present invention are incorporated into oral tablets, such tablets can be compressed, tablet triturates, enteric-coated, sugar-coated, film-coated, multiply compressed or multiply layered. Liquid oral dosage forms include aqueous and non-aqueous solutions, emulsions, suspensions, and solutions and/or suspensions reconstituted from non-effervescent granules, containing suitable solvents, preservatives, emulsifying agents, suspending agents, diluents, sweeteners, coloring agents, and flavoring agents. When the compounds of the present invention are to be injected parenterally, they may be, e.g., in the form of an isotonic sterile solution. Alternatively, when the compounds of the present invention are to be inhaled, they may be formulated into a dry aerosol or may be formulated into an aqueous or partially aqueous solution.

In addition, when the compounds of the present invention are incorporated into oral dosage forms, it is contemplated that such dosage forms may provide an immediate release of the compound in the gastrointestinal tract, or alternatively may provide a controlled and/or sustained release through the gastrointestinal tract.. A wide variety of controlled and/or sustained release formulations are well known to those skilled in the art, and are contemplated for use in connection with the formulations of the present invention. The controlled and/or sustained release may be provided by, e.g., a coating on the oral dosage form or by incorporating the compound(s) of the invention into a controlled and/or sustained release matrix.

Specific examples of pharmaceutically acceptable carriers and excipients that may be used for formulate oral dosage forms, are described in the Handbook of Pharmaceutical Excipients, American Pharmaceutical Association (1986), incorporated by reference herein. Techniques and compositions for making solid oral dosage forms are described in Pharmaceutical Dosage Forms: Tablets (Lieberman, Lachman and Schwartz, editors) 2nd edition, published by Marcel Dekker, Inc., incorporated by reference herein. Techniques and compositions for making tablets (compressed and molded), capsules (hard and soft gelatin) and pills are also described in Remington's Pharmaceutical Sciences (Arthur Osol, editor), 1553-1593 (1980), incorporated herein by reference. Techniques and composition for making liquid oral dosage forms are described in Pharmaceutical Dosage Forms: Disperse Systems, (Lieberman, Rieger and Banker, editors) published by Marcel Dekker, Inc., incorporated herein by reference.

When the compounds of the present invention are incorporated for parenteral administration by injection (e.g., continuous infusion or bolus injection), the formulation for parenteral administration may be in the form of suspensions, solutions, emulsions in oily or aqueous vehicles, and such formulations may further comprise pharmaceutically necessary additives such as stabilizing agents, suspending agents, dispersing agents, and the like. The compounds of the invention may also be in the form of a powder for reconstitution as an injectable formulation.

The dose of the compounds of the present invention is dependent upon the affliction to be treated, the severity of the symptoms, the route of administration, the frequency of the dosage interval, the presence of any deleterious side-effects, and the particular compound utilized, among other things.

The PDE IV inhibitory compounds of the present invention may be examined for their PDE IV inhibitory effects via the techniques set forth in the following.

### 5-chloro-2-(3,5-di-t-butyl-4-hydroxy-benzyl)-7-(2-(2-pyridyl)-ethynyl)-benzoxazole

### (a) 2-bromo-4-chloro-6-nitro-phenol

A solution of2-bromo-4-chloro-phenol (99.24 g, 480 mmol) in acetic acid (110 ml) and acetic anhydride (125 ml) was cooled to -10°C. Within 1 hour a solution containing 100% nitric acid (33 ml) and acetic acid (40 ml) was added between -10° and -5°C, with stirring. The mixture was stirred for an additional 1.5 hours at 0-5°C, then the suspension poured onto 300 g of ice in 700 ml of water and stirred for a further 0.5 hour. The solid was collected, washed, and dried to give 97.12 g (80.1%) of the title compound (mp 121-2°C).

### (b) 6-amino-2-bromo-4-chloro-phenol

A solution of 2-bromo-4-chloro-6-nitro-phenol (16.27 g, 64.4 mmol) in ethyl acetate (160 ml) was hydrogenated, at room temperature, with Raney-nickel (6 g). After hydrogen uptake (approx. 4.8 I) was complete, the nickel was removed by filtration and the filtrate evaporated *in-vacuo* to give 14.19 g (99.0%) of the title compound which was suitable for the next step.

### (c) N,O-di-(3,5-di-t-butyl-4-hydroxy-phenylacetyl)-6-amino-2-bromo-4-chloro-phenol

Water (173 ml) and sodium carbonate (33.24 g, 310 mmol) were added to a stirred ethereal solution (123 ml) of 6-amino-2-bromo-4-chloro-phenol (17.45 g, 78.4 mmol). After 15 minutes 3,5-di-t-butyl-4-hydroxy-phenylacetyl chloride (47.60 g, 93.1%, 156.8 mmol) (prepared with thionyl Chloride from the corresponding acid), was added at -5° to 0°C and stirring continued for a further 1.5 hours without cooling. The aqueous phase was adjusted to pH 8 and the layers separated. The organics were washed with I N HCl (100 ml) and saturated aqueous sodium bicarbonate solution (100 ml), dried (Na₂SO₄) and evaporated *in-vacuo* to give 58.1 g (103.6%) of the title compound which was suitable for the next step.

### (d) 2-bromo-4-chloro-6-(3,5-di-t-butyl-4-hydroxy-phenylacetyl-amino)-phenol

A solution of N,O-Di-(3,5-di-t-butyl-4-hydroxy-phenylacetyl)-6-amino-2-bromo-4-chloro-phenol (58.1 g, 89.8 mmol) in methanol (400 ml) and potassium carbonate (24.78 g, 180 mmol) was stirred at room temperature for 10 minutes. The methanol was removed *in-vacuo*, the residue treated with 2 N HCI (180 ml, 360 mmol), and extracted with ethyl acetate (300 ml). The organics were dried (Na₂SO₄), evaporated *in-vacuo*, and the residue suspended in petroleum ether. The precipitate was collected to give 37.44 g (88.9%) of the title compound which was suitable for the next step.

### (e) 7-bromo-5-chloro-2-(3,5-di-t-butyl-4-hydroxy-benzyl)-benzoxazole

A solution of 2-bromo-4-chloro-6-(3,5-di-t-butyl-4-hydroxy-phenylacetyl-amino)-phenol (35.67 g, 76.1 mmol) and phosphorus oxychloride (41.8 ml, 457 mmol) in toluene was heated under reflux for 1 hour. Volatiles were removed *in-vacuo* and residual amounts of phosphorus oxychloride removed by azeotropic distillation with toluene (2 x 50 ml). The residue was taken up in acetone (50 ml) and ether (100 ml), and treated with water (100 ml) and saturated aqueous sodium bicarbonate solution (100 ml). The organic solvents were removed *in-vacuo* and the precipitate collected to give 33.36 g (93.6%) of crude benzoxazole. The crude benzoxazole was dissolved in dichloromethane (100 ml), filtered, and the filtrate diluted with methanol (100 ml). The dichloromethane was removed by distillation and the resulting crystals collected, washed, and dried *in-vacuo* to give 28,86 g (80.9%) of the title compound (mp 133-6°C).

### (f) 5-chloro-2-(3,5-di-t-butyl-4-hydroxy-benzyl)-7-ethynyl-benzoxazole

A suspension of 7-bromo-5-chloro-2-(3,5-di-t-butyl-4-hydroxy-benzyl)-benzoxazole (13.50 g, 30 mmol), trimethylsilylacetylene (4.41 g, 6.36 ml, 45 mmol), bis(triphenylphosphinc) palladium (II) dichloride (105 mg, 150 µmol) and copper (I) iodide (5.75 mg, 30 µmol) in triethylamine (60 ml) was heated at 90°C, under argon, for 3 hours. The mixture was cooled to room temperature, diluted with water (375 ml) and the excess triethylamine removed *m-vacuo*. The solid was removed by filtration and the filtrate evaporated *m-vacuo* to give 14.00 g (100%) of crude trimethylsilylacetylene derivative. A suspension of the crude trimethylsilylacetylene derivative (14 g) in methanol (140 ml) and potassium carbonate (6.20 g, 45 mmol) was stirred at room temperature, under nitrogen, for 10 minutes, 2 N HCl (45 ml, 90mmol) was added slowly and the formed suspension evaporated *in-vacuo*. The residue was taken up in dichloromethane (200 ml), the salt removed by filtration and the filtrate evaporated *in-vacuo* to give 12.21 g (102.8%) of crude 5-chloro-2-(3,5-di-t-butyl-4-hydroxy-benzyl)-7-ethynyl-benzoxazole. The crude ethynyl-benzoxazole was dissolved in dichloromethane (40 ml) and filtered through 60 g of silica gel. The product was recrystallized from methanol to give 8.10 g (68.2%) of the title compound (mp 152-5°C). From the filtrate a second crop of 1.31 g (11.0%) was also obtained.

| Elemental analysis for C₂₄H₂₆ClNO₂ | | | |
|---|---|---|---|
| Calc. C 72.81 | H 6.62 | N 3.54 | O 8.10 |
| Found C 72.26 | H 6.60 | N 3.72 | O 8.07 |

### (g) 5-chloro-2-(3,5-di-t-butyl-4-hydroxy-benzyl)-7-(2-(2-pyridyl)-ethynyl)-benzoxazole

A suspension of 5-chloro-2-(3,5-di-t-butyl-4-hydroxy-benzyl)-7-ethynyl-benzoxazole (2.38 g, 6.0 mmol), 2-bromo-pyridine (0.66 ml, 98%, 6.6 mmol), bis(triphenylphosphine)palladium(II) dichloride (21.1 mg, 30 µmol) and copper (I) iodide (1.2 mg, 6 µmol) in triethylamine (12 ml) was heated at 90°C, under argon, for 1.5 hours. The triethylamine was removed *in-vacuo* and the residue dissolved in ether (100 ml). The organics were washed with water (50 ml), 1 N HCI (100 ml) and saturated aqueous sodium hydrogen carbonate (100 ml), dried (Na₂SO₄) and evaporated *in-vacuo* to give 2.96 g (104.2%) of crude pyridylethynylbenzoxazole. The crude benzoxazole was purified by column chromatography (SiO₂; dichloromethane), and the product crystallized from methanol and suspended in hot water. The resulting crystals were collected, washed, and dried to give 1.49 g (52.5%) of the title compound (mp 138-9°C).

| Elemental analysis for C₂₉H₂₉CIN₂O₂ | | | |
|---|---|---|---|
| Calc. C 73.64 | H 6.18 | N 5.92 | O 6.76 |
| Found C 73.62 | H 5.97 | N 5.91 | O 6.93 |

### EXAMPLE 2

### 2-((3-cyclopentyloxy-4-methyoxy)benzyl)-4-(2-pyridylmethoxy)-benzoxazole hydrochloride

2-(3-cyclopentyloxy-4-methoxybenzyl)-4-hydroxybenzoxazole (0.99 g, 2.91 mmol) was suspended in acetonitrile (40 ml) containing potassium carbonate (3.12 g, 22.5 mmol), 2-pyridylmethyl chloride (0.62 g, 3.78 mmol) and sodium iodide (41 mg). The resulting mixture was heated at reflux with stirring overnight. The reaction mixture was poured onto water (150 ml) and extracted with ethyl acetate (3 x 100 ml). The combined organic extracts were washed with water (150 ml) and brine (100 ml), dried (CaSO₄) and evaporated *in-vacuo* to afford a brown oil. The residue was purified by flash chromatography (SiO₂; dichloromethane (50:1)) to afford the free base of title compound as a colorless oil (0.772 g, 62%). The product was dissolved in ether (50 ml) and to the resultant solution was added a solution of hydrochloric acid (1 M in ether). The resultant colorless precipitate was collected by filtration, washed with ether (50 ml) and dried *in vacuo* over P₂O₅ at room temperature to afford the title compound (0.747 g, 55%) as a colorless powder (mp 121-125°C).
δ_{H} (250 MHz, d₆ DMSO) 1.52-1.82 (8H,m,4 x CH₂), 3.70 (3H,s,OMe), 4.21 (2H,s,CH₂), 4.73 (1H,m,CH), 5.56 (2H,s,CH₂), 6.82 (1H,dd, ArH), 6.89 (1H,d,ArH), 6.95 (1H,d,ArH), 7.00(1H,m,ArH), 7.23-7.29 (2H,m,ArH), 7.67 (1H,m,ArH), 7.83 (1H,d,ArH), 8.19 (1H,dt,ArH), 8.75 (1H,d,ArH).

### EXAMPLE 3

Protocols for PDE IV, PDE III, and PDE V inhibition activity are set forth below:

### Type III Phosphodiesterase Enzyme Isolation Protocol

The Type III PDE is isolated from human platelets using a procedure similar to that previously described by Washer, R.E. et al., Biochem. Pharmacol., 35:787, 1986. Briefly, 1-2 units of platelets are suspended in an equal volume of buffer (20 mM Tris-HCl, pH 7.5, containing 2 mM magnesium acetate, 1 mM dithiothreitol, and 5 mM Na₂ EDTA). The proteinase inhibitor phenylmethyl-sulfonyl fluoride (PMSF) is also included in this buffer at a final concentration of 200 µM. The suspension is homogenized using a polytron and the homogenate centrifuged at 100,000 x g for 60 minutes. This and all subsequent procedures are performed at 0-4°C. The supernatant is then filtered through four layers of gauze and applied to a DEAE-Trisacryl M column, previously equilibrated with buffer B (20 mM Tris-HCl, pH 7.5, containing 1 mM magnesium acetate, 1 mM dithiothreitol and 200 µM PMSF). After application of the sample, the column is washed with several bed volumes of buffer B, after which the different forms of PDE are eluted from the column using two successive linear NaCl gradients (0.05-0.15 M, 300 ml total; 0.15-0.40 M, 200 ml total). Five ml fractions are collected and assayed for cyclic AMP and cyclic GMP PDE activity. Fractions containing PDE III activity are pooled and dialyzed overnight against 4 L of buffer B. The dialyzed PDE III is then concentrated to 10% of the original volume, diluted to 50% with ethylene glycol monoethyl ether and stored at -20°C. PDE III can typically be retained for up to four weeks with little or no loss of activity.

### Measuring Type III PDE Activity

Enzyme activity is assessed by measuring the hydrolysis of [³H]-cyclic AMP, as described by Thompson. W.J. et al., Adv. Cyclic Nucleotide Res. 10:69, 1979. The cyclic AMP concentration used in this assay is 0.2 µM, which approximates to the Kₘ value. Protein concentration is adjusted to ensure that no more than 15% of the available substrate is hydrolyzed during the incubation period.

All test compounds are dissolved in dimethyl sulfoxide (final concentration of 2.5%). This concentration of dimethyl sulfoxide inhibits enzyme activity by approximately 10%.

### Type IV Phosphodiesterase Enzyme Isolation Protocol

The Type IV PDE is isolated from bovine tracheal smooth muscle using a procedure similar to that previously described by Silver, P.J. et al., Eur. J. Pharmacol. 150:85, 1988 (1). Briefly, smooth muscle from bovine trachea is minded and homogenized using a polytron in 10 volumes of an extraction buffer containing 10 mM Tris-acetate (pH 7.5), 2 mM magnesium chloride, 1 mM dithiothreitol and 2,000 units/ml of aprotinin. This and all subsequent procedures are performed at 0-4°C. The homogenate is sonicated and then centrifuged at 48,000 x g for 30 minutes. The resulting supernatant is applied to a DEAE Trisacryl M column previously equilibrated with sodium acetate and dithiothreitol. After applications of the sample, the column is washed with sodium acetate/dithiothreitol, after which the different forms of PDE are eluted from the column using a linear Tris-HCl/NaCl gradient. Fractions containing Type IV PDE are collected, dialyzed and concentrated to 14% of the original volume. The concentrated fractions are diluted to 50% with ethylene glycol and stored at - 20°C.

### Measuring Type IV PDE Activity

Enzyme activity is assessed by measuring the hydrolysis of [³H]-cyclic AMP, as described by Thompson, W.J. et al., Adv. Cyclic Nucleotide Res. 10:69, 1979. The cyclic AMP concentration used in this assay is 0.2 µM, which approximates the Kₘ value. Protein concentration is adjusted to ensure that no more than 15% of the available substrate is hydrolyzed during the incubation period.

All test compounds are dissolved in dimethyl sulfoxide (final concentration of 2.5%). This concentration of dimethyl sulfoxide inhibits enzyme activity by approximately 10%

### EXAMPLE 4

Following the above procedures, the PDE III, PDE IV inhibition for the compounds of Examples 1 and 2, and rolipram are tested and compared. The results are shown in Table 1 below:

**TABLE I**

| **IC**_{**50**}**(µM)** | | |
|---|---|---|
| **Example** | **PDE III** | **PDE IV** |
| 1 | >300 | 0.08 |
| 1 | >300 | 0.01 |
| 2 | >300 | 0.53 |
| Rolipram | 620 | 3.70 |

### EXAMPLE 5

### 2-((3-Cyclopentyloxy-4-methoxy)benzyl)-7-(2-pyridine-carbonylamino)-benzoxazole

A solution of 1,1'-carbonyl diimidazole (1.45 g, 0.0089 mol) in dichloromethane (10 ml) was added to a suspension of picolinic acid (0.737 g, 0.0060 mol) in dichloromethane (10 ml) under argon at room temperature. As the mixture was stirred the suspended solids dissolved. After 2 hours, this solution was added to a solution of 7-amino-2-((3-cyclopentyloxy-4-methoxy)benzyl)-benzoxazole (1 006 g, 0.0030 mol) and pyridine (0 25 ml) in dichloromethane (10 ml) and the mixture was stirred at room temperature After 6 days the reaction mixture was diluted with water (50 ml) and extracted with dichloromethane (2 x 50 ml) The organic layers were combined, washed with water (2 x 30 ml) and sodium hydrogen carbonate (2 x 50 ml of a saturated solution), dried over calcium sulfate, evaporated *in vacuo* and purified by flash chromatography (SiO₂, petrol/ethyl acetate. 2.1 v/v) to afford the title compound (0.269 g, 0.0006 mol, 20%) as an off white solid (mp 119 4 - 122 3°C. corrected).
δ_{H} (250 MHz; d₆ DNISO) 1.47-1.81 (8H, m, 4 x -CH₂-), 3.71 (311. s, -OCH₃). 4.28 (2H, s, Ar-CH₂-C), 4.75 (1H, m, -CH-), 6.85 (1H, dd, 6-H), 6.90 (1H, d, 5'-H). 7.00 (1H, dd, 2'-H), 7.36 (1H, t, 5-H), 7.52 (1H, d, Ar-H), 7.73 (1H, m, pyridine Ar-H), 7.84 (1H, d, Ar-H), 8.11 (1H, dt, pyridine Ar-H), 8.19 (1H, d, pyridine Ar-H), 8.77 (1H, d, pyridine Ar-H), 10.66 (1H, s, NH)
ν (KBr Disc) 3336, 2961, 2873, 2836, 1685, 1637, 1618, 1590, 1574, 1536, 1512, 1471, 1447, 1434, 1420, 1352, 1340, 1288, 1260, 1234, 1187, 1163, 1144, 1127, 1086, 1049, 1023, 997, 964, 946, 924, 904, 888, 851, 813, 789, 772, 740, 686, 646, 611 cm⁻¹.

### EXAMPLE 6

### 2-((3,5-Di-t-butyl-4-hydroxy)henzyl-7-(2-(2-pyridyl)-ethynyl)-henzoxazole

### (a) 2-Bromo-6-(3,5-di-t-butyl-4-hydroxyphenyl)acetylamido-phenol

(3,5-Di-*t*-butyl-4-hydroxyphenyl)acetic acid (5.03 g, 0.019 mol) in dichloromethane (20 ml) was added dropwise over 0.25 hours to a stirred solution of 1,1'-carbonyl diimidazole (4.68 g, 0.029 mol) in dichloromethane (60 ml). After 2 hours, this solution was added dropwise over 0.5 hours to a solution of6-amino-2-bromophenol (4.30 g of a mixture containing 6-amino-2-di-bromophenol (3.60 g, 0.019 mmol), and 6-amino-2,6-di-bromophenol (0.70 g, 0.003 mol)) in dichloromethane (30 ml) After 18 hours, the reaction mixture was diluted with ethyl acetate (200 ml) and washed with 2 M hydrochloric acid (100 ml), and water, dried over magnesium sulfate, evaporated *in vacuo*, and purified by flash chromatography (SiO₂, dichloromethane) to furnish a 4 1 w/w (as judged by ¹H NMR) mixture of 2-bromo-6-(3,5-di-*t*-butyl-4-hydroxyphenyl)acetylamido-phenol and 2,4-di-bromo-6-(3,5-di-t-butyl-4-hydroxyphenyl)acetylamido-phenol (6.90 g) as a pale pink solid. δ_{H} (250 MHz; d₆ DMSO) 1.37 (18 H, s, 2 x (-CH₃)₃), 3.62 (2 H, s, Ar-CH₂-), 6.78 (1 H, dd, Ar-H), 6.88 (1 H, s, -OH) 7.09 (2 H, s, 2'-,6'-H), 7.33 (1 H, dd, Ar-H), 7.43 (1 H, dd, 6-H), 9.92 (1 H, bs. -NH-), 10.02 (1 H, s, -OH).
A signal at δ = 7.51 ppm (d) was attributable to 2,4-di-bromo-6-(3,5-di-*t*-butyl-4-hydroxyphenyl)-acetylanido-phenol.

### (b) 7-Bromo-2-((3,5-di-t-butyl-4-hydroxy)benzyl)-benzoxazole

2-Bromo-6-(3,5-di-*t*-butyl-4-hydroxyphenyl)acetylamido-phenol (6.90 g, 80%, 0.013 mol) and pyridinium toluenesulfonate (1.37 g, 0.0055 mol) were suspended in xylene (165 ml) and heated to reflux. After 17 hours, the reaction mixture was allowed to cool, diluted with water (200 ml) and extracted with ethyl acetate (3 x 100 ml). The organic extracts were combined, dried over magnesium sulfate, evaporated *in vacuo* and purified by flash chromatography (SiO₂, dichloromethane/petrol 2:1 v/v) to afford the title compound (4.90 g, 0.0015 mol, 92%) as an off-white solid.
δ_{H} (250 MHz; d₆ DMSO) 1.35 (18 H, s, 2 x (-CH₃)₃), 4.24 (2 H, s, Ar-CH₂-), 6.95 (1 H, s, -OH) 7.14 (2 H, s, 2'-,6'-H), 7.28 (1 H, dd, 5-H), 7.57 (1 H, dd, 6-H), 7.69 (1 H, dd, Ar-H).

### (c) 2-((3,5-Di-t-butyl-4-hydroxy)benzyl-7-(2-(2-pyridyl)-ethynyl)-benzoxazole

Argon was bubbled through a mixture of 7-bromo-2-((3,5-di-t-butyl-4-hydroxy)benzyl)-benzoxazole, (1.002 g, 0.0024 mol), copper(I) iodide (2 mg), and 2-ethynylpyridine (0.320 ml, 0.326 g, 0.0032 mol) in triethylamine (6 ml). After 0.75 hours bis(triphenylphosphinc)palladium(II) dichloride (0.045 g, 0.00006 mol) was added and the reaction mixture was flushed with argon for a further 5 minutes then heated to 90°C. After 1.5 hours, a new product was observed by TLC (SiO₂, petrol/diethyl ether 1:1 v/v) together with both starting materials. Further portions of 2-ethynylpyridine were added after 2 hours (0.100 ml, 0.102 g, 0.0099 mol) and 4.5 hours (0.200 ml, 0.204 g, 0.0020 mol). No change was observed by TLC. An additional portion of bis(triphenylphosphine)palladium(II) dicliloride (0.02 g. 0.00003 mol) was also added after 4.5 hours and the mixture was stirred at 90°C for a further 3 hours and at ambient temperature for a further 16 hours. No change was observed by TLC. The reaction mixture was evaporated *in vacuo* and purified by flash chromatography (SiO₂, petrol/ether 1:1 v/v) to furnish the title compound (0.225 g, 0.00051 mol, 21%) as a dry khaki foam.
δ_{H} (250 MHz; d₆ DMSO) 1.35 (18 H, s. 2 x -C(CH₃)₃), 4.27 (2 H, s, Ar-CH₂), 6.89 (1 H, bd, -OH), 7.17 (2 H, s, 2'-H and 6'-H), 7.41 (1 H, dd, 5-H), 7.47 (1 H, m, Py-H), 7.60 (1 H, d, Ar-H), 7.68. (1 H, d, Py-H), 7.76 (1 H, d, Ar-H), 7.89 (1 H, m, Py-H), 8.64 (1 H, m, Py-H).
ν (KBr Disc) 3633, 3450, 2956, 2927, 2912, 2871, 2221, 1604, 1582, 1564, 1488, 1462, 1424, 1400, 1390, 1361, 1315, 1279, 1259, 1236, 1212, 1189, 1148, 1136, 1121, 1095, 1048, 1036, 989, 822, 795, 776, 741 cm⁻¹.

### EXAMPLE 7

### 2-((3,5-Di-isopropyl-4-hydroxy)-benzyl-7-(2-(2-pyridyl)-ethynyl)-benzoxazole

### (a) 2-Bromo-6-(3,5-di-isopropyl-4-hydroxyphenyl)acetylamido-phenol

(3,5-Di-isopropyl-4-hydroxyphenyl)acetic acid (6.28 g, 0.027 mol) in dichloromethane (40 ml) was added to a stirred solution of 1,1'-carbonyl diimidazole (6.50 g, 0.04 mol) in dichloromethane (40 ml). After 1 hour, a solution of 6-amino-2-bromophenol (4.0 g of a mixture containing 6-amino-2-di-bromophenol (3.35 g, 0.018 mmol), and 6-amino-2,6-di-bromophenol (0.65 g, 0.0024 mol)) in dichloromethane (25 ml) was added and the mixture was stirred at room temperature. After 20 hours, the mixture was washed with 1 M hydrochloric acid (2 x 80 ml), evaporated *in vacuo*, and purified by flash chromatography (SiO₂, petrol/ether 1:1 v/v, ether, and dichloromethane/methanol 20:1 v/v) to furnish a 17:3 w/w (as judged by ¹H-NMR) mixture of 2-bromo-6-(3,5-di-isopropyl-4-hydroxyphenyl)acetylamido-phenol and 2,4-di-bromo-6-(3,5-d-isopropyl-4-hydroxyphenyl)acetylamido-phenol (5.70 g) as a yellow solid.
δ_{H} (250 MHz; CDCl₃) 1.25 (12H, d, 2 x -CH(CH₃)₂). 3.15(2 H, m, 2 x -CH(CH₃)₂), 3.71 (2 H, s, ArCH₂-), 4.87 (1 H, s, -OH), 6.71 (1 H, t. 5-H), 6.98 (2 H, s, 2'-,6'-H), 7.25 (1 H, dd, Ar-H), 7.41 (H, dd, Ar-H), 7.53 (1 H, bs, -NH-), 7.65 (1 H, s, Ar-OH). Two signals at δ = 7.36 (d) ppm and 7.77 (d) are attributable to 2,4-di-bronio-6-(3,5-di-isopropyl-4-hydroxyphenyl)acetylamido-phenol.

### (b) 7-Bromo-2-(3,5-di-isopropyl-4-hydroxy-benzyl)-benzoxazole

2-Bromo-6-(3,5-di-isopropyl-4-hydroxyphenyl)acetylamido-phenol (2.97 g, 85%, 0.065 mol) and pyridinium p-toluenesulfonate (0.612 g. 0.0024 mol) were suspended in xylene (85 ml) and heated to reflux. After 16 hours, the reaction mixture was allowed to cool, diluted with water (140 ml) and extracted with ethyl acetate (2 x 70 ml). The organic extracts were combined, washed with water (70 ml), and brine (70 ml), dried over magnesium sulfate, evaporated *in vacuo* and purified by flash chromatography (SiO₂, dichloromethane) to afford the title compound (1.44 g, 0.0039 mol, 60%) as a yellow solid.
δ_{H} (250 MHz; CDCl3) 1.28 (12H, d, 2 x -CH(CH₃)₂), 3.15 (2 H, m, 2 x -CH(CH₃)₂), 4.22; (2 H, s, ArCH₂-), 4.87 (1 H, s, -OH), 7.13 (2 H, s, 2'-,6'-H), 7.19 (1 H, t, 5-H), 7.44 (1 H, d, Ar-H), 7.61 (1 H, d, Ar-H).

### (c) 2-((3,5-di-isopropyl-4-hydroxy)benzyl-7-(2-(2-pyridyl)-ethynyl)-benzoxazole

Argon was bubbled through a mixture of 7-bromo-2-((3,5-di-isopropyl-4-hydroxy)benzyl)-benzoxazole, (0.785 g, 0.0020 mol), copper(I) iodide (4 mg), and 2-ethynylpyridine (0.400 ml, 0.408 g, 0.0040 mol) in triethylamine (12 ml). After 0.5 hours bis(triphenylphosphine)palladium(II) dichloride (0.074 g, 0.0001 mol) was added and the reaction mixture was flushed with argon for a further 5 minutes then heated to 90°C. After 16 hours, the reaction mixture was evaporated *in vacuo* and purified by flash chromatography (SiO₂, petrol/ether 1:1 v/v) and crystallized from ether to furnish the title compound (0.318 g, 0.00078 mol, 38%) as a green crystalline solid (mp 145.4-148.8°C).
δ_{H} (250 MHz; d₆ DMSO) 1.10 (12H, d, 2 x CH(CH₃)₂), 3.25 (2 H, m,2 x CH(CH₃)₂) 4.25 (2H,s, -CH₂-), 7.03 (2H, s, 2'-H and 6'-H), 7.39 (1H, t, 5-H), 7.45 (1H, m, Py-H), 7.59 (1H, d, Ar-H), 7.67 (1H, d, Py-H), 7.80 (1H, d, Ar-H), 7.89 (1H, dt, Py-H), 8.04 (1H, s, Ar-OH), 8.64 (1H, bd, Py-H)
ν (KBr Disc) 3280, 2958, 2927, 2868, 2219, 1731, 1721, 1706, 1702, 1691, 1687, 1676, 1591, 1581, 1562, 1463, 1443, 1423, 1319, 1292, 1281, 1260, 207, 1157, 1145, 1122, 1047, 795, 777, 740 cm⁻¹.

### EXAMPLE 8

Following the procedures set forth in Examples 3 and 4. the PDE III and PDE IV inhibition for the compounds of Examples 5, 6 and 7 was calculated and compared to rolipram. The results are shown the Table II below expressed as IC₅₀ values.

**TABLE II**

| IC₅₀ (µM) | | |
|---|---|---|
| EXAMPLE | PDE III | PDE IV |
| 5 | 121.4 | 0.76 |
| 6 | >300 | 0.07 |
| 7 | 152.2 | 0.26 |

As was the case with the first group of compounds tested, these inventive compounds also provide high levels of PDE-IV inhibition while at the same time relatively low levels of PDE-III inhibition.

### EXAMPLE 9

### 2-(3-chlorophenyl-7-(2-pyridylmethylamino)benzoxazole

### (a) N-(3-chlorobenzoyl)-2-hydroxy-3-nitroaniline

A stirred solution of 1,1'-carbonyl diimidazole (1.11 g, 0.0068 mol) in 20 ml of tetrahydrofuran was treated with 3-chlorobenzoic acid (1.02 g, 0.0065 mol). After stirring for 1 hour, 2-hydroxy-3-nitroaniline (1.00 g, 0.0065 mol) was added and the reaction mix was allowed to stir overnight. The next day, water (100 ml) was added and the aqueous suspension was extracted with CH₂Cl₂(2 x 100 ml). The CH₂Cl₂ layers were separated, washed with 2 x 50 ml water, dried and evaporated to give 1.92 g of yellow solid. This material was recrystallized from 150 ml of methanol and dried to give 1.80 g of title compound.
'H NMR (60 MHz, CDCl₃) δ 6.44-7.48 (6H,m,ArH), 8.16 (1H,bs,NH or OH) 8.37 (1H,d,ArH), 9.00 (1H,bs,NH or OH).

### (b) 2-(3-chlorophenyl)-7-nitrobenzoxazole

A stirred suspension of N-(3-chlorobenzoyl)-2-hydroxy-3-nitroaniline (4.5 g, 0.0117 mol), p-toluenesulfonic acid (0.30 g) and 50 ml of diphenyl ether were heated at 190 °C for 1 hour. The dark reaction mixture was dissolved in 100 ml CH₂Cl₂ and washed with saturated sodium bicarbonate and then water. The CH₂Cl₂ was evaporated and hexane was added, causing a brown solid to separate. The dark solid was filtered, dissolved in 75% CH₂Cl₂ in hexane and washed onto a silica gel column. Elution with this solvent, gave 3.3 g of the title compound. This material could be recrystallized from methanol to give 2-(3-chlorophenyl)-7-nitrobenzoxazole although it was pure enough from the chromatography to use for the next step without any further purification.
¹HMR (60 MHz, CDCl₃) δ 6.59-7.63 (7H,m,ArH).

### (c) 2-(3-chlorophenyl)-7-aminobenzoxazole

A suspension of 2-(3-chlorophenyl)-7-nitrobenzoxazole (3.3 g. 0.012 mol) and 5 g of a 50 % dispersion of Raney nickel in 130 ml of ethanol, was heated to 50 °C on a steam bath. The reaction temperature was held at 50 °C as a blend of 23 ml anhydrous hydrazine, 15 ml water and 20 ml of ethanol was added (30 minutes). The steam bath was removed and the reaction mixture was stirred at room temperature for 2.5 hours. The Raney nickel was removed by filtration through celite and the aqueous ethanol solution was concentrated on a rotary evaporator under reduced pressure. Water (100 ml) and CH₂Cl₂ (150 ml) were added to the residue and the CH₂Cl₂ layer was separated. The aqueous layer was extracted with 150 ml of CH₂Cl₂ and the combined CH₂Cl₂ layers were washed with 2 x 100 ml of water, dried and evaporated to give 2.90 g of yellow solid. This material was dissolved in 300 ml ethanol and filtered. The filtrate was concentrated to 100 ml and allowed to crystallize at freezer temperatures, to give the title compound as an off-white solid, mp 143-144°C, (1.97 g). A further 0.42 g of the title compound was obtained. by evaporation of the mother liquor from the recrystallization and flash chromatography on silica gel using a 50:50 blend of CH₂Cl₂ in hexane as the eluent.
¹H NMR (60 MHz, CDCl₃) δ 3.84 (2H,bs,NH₂), 6.23-7.78 (7H, m, ArH).

### 2-(3-chlorophenyl)-7-(2-pyridylmethylamino)henzoxazole

(d) To a stirred suspension of 2-(3-chlorophenyl)-7-amino-benzoxazole (1.10 g, 0.0045 mol) in 10 ml of methanol, 2.5 ml of 1 N HCl in methanol, was added. 2-pyridinecarboxaldehyde(0.043 g, 0.0040 mol) was added to this suspension followed by sodium cyanoborohydride (0.32 g, 0.0051 mol) and the pH was adjusted to 6 using 1 N HCl in methanol. After 1 hour, 1 N sodium hydroxide was added to bring the pH to 10 and the reaction was extracted with 3 x 50 ml of ethyl acetate. The combined ethyl acetate extractions were washed with water, dried and evaporated to give 1.3 g of yellow solid. This solid was purified by flash chromatography over silica gel using 50:50 CH₂Cl₂ in hexane to give 0.25 g of recovered starting material, followed by 0.90 g of product when the eluting solvent was CH₂Cl₂. This solid was recrystallized from 100 ml of hexane to give 0.78 g of the title compound, mp 115-116 °C.
¹H NMR(60 MHz, CDCl₃) δ 4.34 (2H,s,CH₂) 4.90 (1H,bs,NH) 6.23 (1H,q,ArH) 6.70-7.70 (9H,m,ArH), 8.18 (1H,d,ArH).

### EXAMPLE 10

Following the procedures set forth in Examples 3 and 4, the PDE III and PDE IV inhibition values for the compounds of Example 9 was calculated. The result are shown the Table III below expressed as IC₅₀ value

**TABLE III**

| IC₅₀(µM) | | |
|---|---|---|
| EXAMPLE | PDE III | PDE IV |
| 9 | 173.2 | 9.10 |

As can be seen from the foregoing, the inventive compound provides high levels of PDE IV inhibition while at the same time relatively low levels of PDE-III inhibition. The PDE IV IC₅₀ values has below that of theophylline and

While the invention has been illustrated with respect to the production and use of a particular compound, it was apparent that variations and modifications of the invention can be made.

## Claims

1. A compound of the formula: 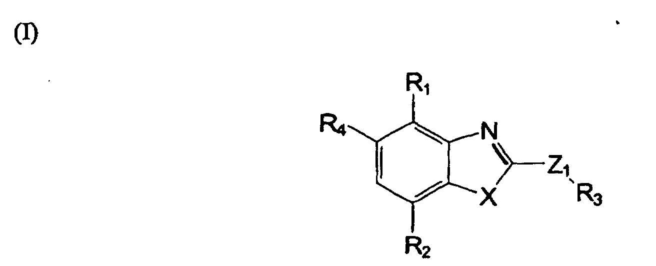 wherein:
X is O or S;
R₁ and R₂ are independently selected from hydrogen, halogen, hydroxy, nitro, QZ₂, OQZ₂, OCOQZ₂, NHQZ₂ or NHCOQZ₂ wherein:
Q is a bond, a straight-chain or branched alkylene, alkenylene or alkynylene group containing from 1 to 12 carbon atoms;
Z₂ is hydrogen, CH(OH)QH, OQH, NO₂, N(QH)₂, CO₂QH, CON(QH)₂, CON(OH)QH, OCOQH, OCON(QH)₂, OCON(OH)QH, NHCON(QH)₂, N(OH)CON(QH)₂, CH=NOCOQH, CH=NOCON(QH)₂, COQH, N(OH)COQH, or a 2-pyridyl being unsubstituted or further substituted with one or more halogen atoms, OH, OQH, NO₂, NH, CO₂QH, CON(QH)₂, OCOQH, and CON(QH)₂;
R₃ is an unsubstituted phenyl or phenyl substituted with 1-3 members independently chosen from the group consisting of OH, halogen, NH₂, NO₂, R₅ or R₆;
Z₁ is a linkage selected from a bond, -CH₂-, -CH=CH-, -CH₂CH₂-, -CH(CH₃)-and -C(CH₃)₂-; except that Z₁R₃ is not 3,5-di-t-butyl-4-hydroxy-phenyl;
R₄ is hydrogen or a halogen;
R₅ is a branched or straight chain alkyl group of 1 to 12 carbon atoms; and
R₆ is a cycloalkyl group of 1 to 12 carbon atoms;
provided that one or both of R₁ and R₂ are QZ₂ wherein Q is an alkenylene or alkynylene and Z₂ is a 2-pyridine.

2. A compound according to claim 1, wherein one of R₁ and R₂ is hydrogen; X is O and R₄ is halogen.

3. A compound according to claim 1, wherein Z₁ is selected from the group consisting of -CH₂-, -CH₂CH₂- and -CH=CH-.

4. A compound according to claim 3, wherein R₄ is chlorine.

5. A compound according to claim 1, wherein Q is an alkenylene.

6. A compound according to claim 1, wherein Q is an alkynylene.

7. A compound according to claim 1, wherein Q is an alkynylene and Z₂ is a pyridine.

8. A compound according to claim 1, wherein Q is an alkynylene and Z₂ is a thiazole.

9. A compound according to claim 1, wherein R₃ is selected from the group consisting of unsubstituted and substituted phenyls.

10. A compound according to claim 9, wherein said phenyl is selected from the group consisting of chlorophenyls, fluorophenyls and chloro-fluorophenyls.

11. A compound according to claim 9, wherein R₃ is 3-cyclopentyloxy-4-methoxyphenyl or 3,4-dimethoxyphenyl.

12. A compound according to claim 9, wherein R₃ is selected from the group consisting of 3,5-di-t-butyl-4-hydroxyphenyl; and 3,5-di-t-butyl-4-acetyloxyphenyl.

13. A compound according to claim 5, wherein said alkenylene is -CH=CH-, -CH₂-CH=CH- or -CH=CH-CH₂-.

14. A compound according to claim 6, wherein alkynylene is -C≡C- or -C≡C-CH₂-.

15. A compound according to claim 1, wherein Q is an alkylene group.

16. A compound according to claim 15, wherein said alkylene is -CH₂-, -CH₂-CH₂-or -CH₂-CH₂-CH₂-.

17. A compound according to claim 1, wherein R₅ is a branched or straight chain alkyl group of 1-12 carbon atoms.

18. A compound according to claim 17, wherein R₅ is methyl or ethyl.

19. A compound of claim 1, selected from the group consisting of:
5-chloro-2-(3,5-di-t-butyl-4-hydroxy-benzyl)-7-(2-(2-pyridyl)-ethynyl)-benzoxazole,
2-((3-5-di-t-butyl-4-hydroxy)benzyl)-7-(2-(2-pyridyl)-ethynyl)-benzoxazole, and
2-((3-5-di-isopropyl-4-hydroxy)benzyl)-7-(2-(2-pyridyl)-ethynyl)-benzoxazole.

20. The use of a compound according to any preceding claim in the preparation of a medicament for a method of effecting selective PDE IV inhibition to a patient requiring the same, which comprises administering an effective amount of the compound.

21. The use of claim 20, wherein said compound is selected from the group consisting of:
5-chloro-2-(3,5-di-t-butyl-4-hydroxy-benzyl)-7-(2-(2-pyridyl)-ethynyl)-benzoxazole,
2-((3-5-di-t-butyl-4-hydroxy)benzyl-7-(2-(2-pyridyl)-ethynyl)-benzoxazole, and
2-((3-5-di-isopropyl-4-hydroxy)benzyl-7-(2-(2-pyridyl)-ethynyl)-benzoxazole.

22. A pharmaceutical composition comprising a compound having the chemical structure set forth in claim 1.

23. The pharmaceutical composition of claim 22, wherein said compound is selected from the group consisting of:
5-chloro-2-(3,5-di-t-butyl-4-hydroxy-benzyl)-7-(2-(2-pyridyl)-ethynyl)-benzoxazole,
2-((3-5-di-t-butyl-4-hydroxy)benzyl-7-(2-(2-pyridyl)-ethynyl)-benzoxazole, and
2-((3-5-di-isopropyl-4-hydroxy)benzyl-7-(2-(2-pyridyl)-ethynyl)-benzoxazole.

24. The use of a compound according to any of claims 1 to 19 in the preparation of a medicament for a method of treating a mammal suffering from a disease state selected from the group consisting of asthma, allergies, inflammation, depression, dementia, atopic diseases, rhinitis and disease states associated with abnormally high physiological levels of a member of the group consisting of cytokines, inflammatory cytokines and chemokines, comprising administering an effective amount of the compound.

25. The use of claim 24, wherein said composition is selected from the group consisting of:
5-chloro-2-(3,5-di-t-butyl-4-hydroxy-benzyl)-7-(2-(2-pyridyl)-ethynyl)-benzoxazole,
2-((3-5-di-t-butyl-4-hydroxy)benzyl-7-(2-(2-pyridyl)-ethynyl)-benzoxazole, and
2-((3-5-di-isopropyl-4-hydroxy)benzyl-7-(2-(2-pyridyl)-ethynyl)-benzoxazole.

26. A compound according to claim 1 having a PDE IV IC₅₀ of less than 10µM.

## Patentansprüche

1. Verbindung der Formel: 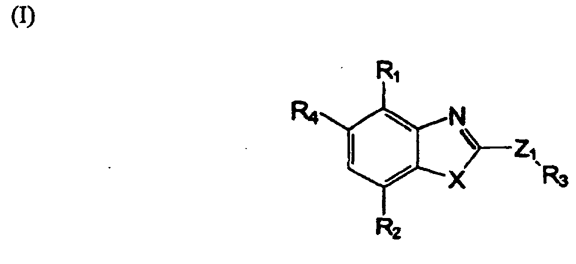 worin:
X für O oder S steht
R₁ und R₂ unabhängig aus Wasserstoff, Halogen, Hydroxy, Nitro, QZ₂, OQZ₂, OCOQZ₂, NHQZ₂ oder NHCOQZ₂ ausgewählt sind, worin:
Q eine Bindung, eine geradkettige oder verzweigte Alkylen-, Alkenylen- oder Alkynylen-Gruppe, enthaltend von 1 bis 12 Kohlenstoffatom(en) ist;
Z₂ Wasserstoff, CH(OH)QH, OQH, NO₂, N(QH)₂, CO₂QH, CON(QH)₂, CON(OH)QH, OCOQH, OCON(QH)₂, OCON(OH)QH, NHCON(QH)₂, N(OH)CON(QH)₂, CH=NOCOQH, CH=NOCON(QH)₂, COQH, N(OH)COQH oder ein 2-Pyridyl, das nicht substituiert oder weiter substituiert mit einem oder mehreren Halogenatom(en), OH, OQH, NO₂, NH, CO₂QH, CON(QH)₂, OCOQH und CON(QH)₂ ist, darstellt;
R₃ ein nicht substituiertes Phenyl oder Phenyl substituiert mit 1-3 Gliedern, unabhängig ausgewählt aus der Gruppe, die aus OH, Halogen, NH₂, NO₂, R₅ oder R₆ besteht, darstellt.
Z₁ eine Verknüpfung, ausgewählt aus einer Bindung, -CH₂-, -CH=CH-, -CH₂CH₂-, -CH(CH₃)- und -C(CH₃)₂- darstellt, außer dass Z₁R₃ nicht 3,5-Di-t-butyl-4-hydroxy-phenyl ist;
R₄ Wasserstoff oder ein Halogen ist;
R₅ eine verzweigte oder geradkettige Alkylgruppe aus einem bis 12 Kohlenstoffatom(en) ist; und
R₆ eine Cycloalkyl-Gruppe aus 1 bis 12 Kohlenstoffatom(en) ist;
vorausgesetzt, dass eines oder beide von R₁ und R₂ für QZ₂ stehen, worin Q ein Alkenylen oder Alkynylen ist und Z₂ ein 2-Pyridin ist.

2. Verbindung nach Anspruch 1, worin eines von R₁ und R₂ Wasserstoff ist; X für O steht und R₄ Halogen ist.

3. Verbindung nach Anspruch 1, worin Z₁ aus der Gruppe, bestehend aus -CH₂-, -CH₂CH₂- und -CH=CH- ausgewählt ist.

4. Verbindung nach Anspruch 3, worin R₄ Chlor ist.

5. Verbindung nach Anspruch 1, worin Q ein Alkenylen ist.

6. Verbindung nach Anspruch 1, worin Q ein Alkynylen ist.

7. Verbindung nach Anspruch 1, worin Q ein Alkynylen ist und Z₂ ein Pyridin ist.

8. Verbindung nach Anspruch 1, worin Q ein Alkynylen ist und Z₂ ein Thiazol ist.

9. Verbindung nach Anspruch 1, worin R₃ aus der Gruppe, bestehend aus nicht substituierten und substituierten Phenylen ausgewählt ist.

10. Verbindung nach Anspruch 9, worin das Phenyl aus der Gruppe, bestehend aus Chlorophenylen, Fluorophenylen und Chloro-fluorophenylen ausgewählt ist.

11. Verbindung nach Anspruch 9, worin R₃ 3-Cyclopentyloxy-4-methoxyphenyl oder 3,4-Dimethoxyphenyl ist.

12. Verbindung nach Anspruch 9, worin R₃ aus der Gruppe, bestehend aus 3,5-Di-t-butyl-4-hydroxyphenyl und 3,5-Di-t-butyl-4-acetyloxyphenyl ausgewählt ist.

13. Verbindung nach Anspruch 5, worin das Alkenylen -CH=CH-, -CH₂-CH=CHoder -CH=CH-CH₂- ist.

14. Verbindung nach Anspruch 6, worin Alkynylen -C≡C- oder -C≡C-CH₂- ist.

15. Verbindung nach Anspruch 1, worin Q eine Alkylen-Gruppe ist.

16. Verbindung nach Anspruch 15, worin das Alkylen -CH₂-, -CH₂-CH₂- oder -CH₂-CH₂-CH₂- ist.

17. Verbindung nach Anspruch 1, worin R₅ eine verzweigte oder geradkettige Alkyl-Gruppe aus 1-12 Kohlenstoffatom(en) ist.

18. Verbindung nach Anspruch 17, worin R₅ Methyl oder Ethyl ist.

19. Verbindung nach Anspruch 1, die aus der Gruppe ausgewählt ist, bestehend aus:
5-Chlöro-2-(3,5-di-t-butyl-4-hydroxy-benzyl)-7-(2-(2-pyridyl)-ethynyl)-benzoxazol,
2-((3-5-Di-t-butyl-4-hydroxy)benzyl)-7-(2-(2-pyridyl)-ethynyl)-benzoxazol und
2-((3-5-Di-isopropyl-4-hydroxy)benzyl)-7-(2-(2-pyridyl)-ethynyl)-benzoxazol.

20. Verwendung einer Verbindung nach einem der vorangehenden Ansprüche bei der Herstellung eines Medikamentes für ein Verfahren zur Bewirkung einer selektiven PDE-IV-Inhibition an einem Patienten, bei dem dies erforderlich ist, das die Verabreichung einer wirksamen Menge der Verbindung umfasst.

21. Verwendung nach Anspruch 20, worin die Verbindung aus der Gruppe ausgewählt ist, bestehend aus:
5-Chloro-2-(3,5-di-t-butyl-4-hydroxy-benzyl)-7-(2-(2-pyridyl)-ethynyl)-benzoxazol,
2-((3-5-Di-t-butyl-4-hydroxy)benzyl-7-(2-(2-pyridyl)-ethynyl)-benzoxazol und
2-((3-5-Di-isopropyl-4-hydroxy)benzyl-7-(2-(2-pyridyl)-ethynyl)-benzoxazol.

22. Pharmazeutische Zusammensetzung, umfassend eine Verbindung mit der in Anspruch 1 dargelegten chemischen Struktur.

23. Pharmazeutische Zusammensetzung nach Anspruch 22, worin genannte Verbindung aus der Gruppe ausgewählt ist, bestehend aus:
5-Chloro-2-(3,5-di-t-butyl-4-hydroxy-benzyl)-7-(2-(2-pyridyl)-ethynyl)-benzoxazol,
2-((3-5-Di-t-butyl-4-hydroxy)benzyl-7-(2-(2-pyridyl)-ethynyl)-benzoxazol und
2-((3-5-Di-isopropyl-4-hydroxy)benzyl-7-(2-(2-pyridyl)-ethynyl)-benzoxazol.

24. Verwendung einer Verbindung nach einem der Ansprüche 1 bis 19 bei der Herstellung eines Medikamentes für ein Verfahren zur Behandlung eines Säugers, der an einem Krankheitszustand leidet, der aus der Gruppe ausgewählt ist, bestehend aus Asthma, Allergien, Entzündung, Depression, Demenz, atopischen Erkrankungen, Rhinitis und Krankheitszuständen, die von abnorm hohen physiologischen Spiegeln eines Gliedes der Gruppe, bestehend aus Cytokinen, inflammatorischen Cytokinen und Chemokinen begleitet sind, das die Verabreichung einer wirksamen Menge der Verbindung umfasst.

25. Verwendung nach Anspruch 24, worin genannte Zusammensetzung aus der Gruppe ausgewählt ist, bestehend aus:
5-Chloro-2-(3,5-di-t-butyl-4-hydroxy-benzyl)-7-(2-(2-pyridyl)-ethynyl)-benzoxazol,
2-((3-5-Di-t-butyl-4-hydroxy)benzyl-7-(2-(2-pyridyl)-ethynyl)-benzoxazol und
2-((3-5-Di-isopropyl-4-hydroxy)benzyl-7-(2-(2-pyridyl)-ethynyl)-benzoxazol.

26. Verbindung nach Anspruch 1 mit einer IC₅₀ von PDE-IV von weniger als 10 µM.

## Revendications

1. Composé de formule: 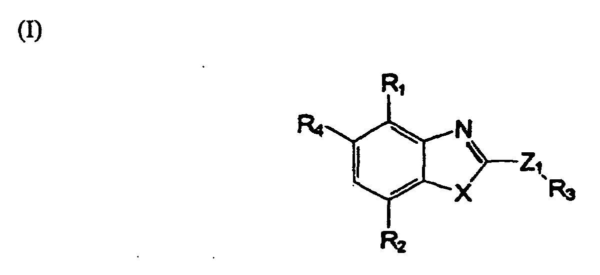 dans laquelle:
X est O ou S;
R₁ et R₂ sont indépendamment choisis parmi un hydrogène, un halogène, un groupe hydroxy, nitro, QZ₂, OQZ₂, OCOQZ₂, NHQZ₂ ou NHCOQZ₂ où:
Q est une liaison, un groupe alkylène, alcénylène ou alcynylène à chaîne droite ou ramifié, contenant 1 à 12 atomes de carbone;
Z₂ est un hydrogène, CH(OH)QH, OQH, NO₂, N(QH)₂, CO₂QH, CON(QH)₂, CON(OH)QH, OCOQH, OCON(QH)₂, OCON(OH)QH, NHCON(QH)₂, N(OH)CON(QH)₂, CH=NOCOQH, CH=NOCON(QH)₂, COQH, N(OH)COQH, ou un groupe 2-pyridyle non substitué ou substitué encore par un ou plusieurs atomes d'halogène, groupes OH, OQH, NO₂, NH, CO₂QH, CON(QH)₂, OCOQH, et CON(QH)2;
R₃ est un groupe phényle non substitué ou un groupe phényle substitué par 1-3 membres indépendamment choisis dans le groupe constitué de OH, un halogène, NH₂, NO₂, R₅ ou R₆;
Z₁ est une liaison choisie parmi une liaison, -CH₂-, -CH=CH-, -CH₂CH₂-, - CH(CH₃)- et -C(CH₃)₂-; à ceci près que Z₁R₃ n'est pas un groupe 3,5-di-t-butyl-4-hydroxyphényle;
R₄ est un hydrogène ou un halogène;
R₅ est un groupe alkyle à chaîne droite ou ramifiée de 1 à 12 atomes de carbone; et
R₆ est un groupe cycloalkyle de 1 à 12 atomes de carbone;
à condition qu'un des R₁ et R₂ ou les deux soient QZ₂ où Q est un groupe alcénylène ou alcynylène et Z₂ est une 2-pyridine.

2. Composé selon la revendication 1, dans lequel un des R₁ et R₂ est un hydrogène; X est O et R₄ est un halogène.

3. Composé selon la revendication 1, dans lequel Z₁ est choisi dans le groupe constitué de -CH₂-, -CH₂CH₂- et -CH=CH-.

4. Composé selon la revendication 3, dans lequel R₄ est un chlore.

5. Composé selon la revendication 1, dans lequel Q est un groupe alcénylène.

6. Composé selon la revendication 1, dans lequel Q est un groupe alcynylène.

7. Composé selon la revendication 1, dans lequel Q est un groupe alcynylène et Z₂ est une pyridine.

8. Composé selon la revendication 1, dans lequel Q est un groupe alcynylène et Z₂ est un thiazole.

9. Composé selon la revendication 1, dans lequel R₃ est choisi dans le groupe constitué des groupes phényle non substitués et substitués.

10. Composé selon la revendication 9, dans lequel ledit groupe phényle est choisi dans le groupe constitué des groupes chlorophényle, fluorophényle et chloro-fluorophényle.

11. Composé selon la revendication 9, dans lequel R₃ est un groupe 3-cyclopentyloxy-4-méthoxyphényle ou 3,4-diméthoxyphényle.

12. Composé selon la revendication 9, dans lequel R₃ est choisi dans le groupe constitué des groupes 3,5-di-t-butyl-4-hydroxyphényle; et 3,5-di-t-butyl-4-acétyloxyphényle.

13. Composé selon la revendication 5, dans lequel ledit groupe alcénylène est - CH=CH-, -CH₂-CH=CH- ou -CH=CH-CH₂-.

14. Composé selon la revendication 6, dans lequel le groupe alcynylène est -C≡C- ou -C≡C-CH₂-.

15. Composé selon la revendication 1, dans lequel Q est un groupe alkylène.

16. Composé selon la revendication 15, dans lequel ledit groupe alkylène est -CH₂-, - CH₂-CH₂- ou -CH₂-CH₂-CH₂-.

17. Composé selon la revendication 1, dans lequel R₅ est un groupe alkyle à chaîne droite ou ramifiée de 1-12 atomes de carbone.

18. Composé selon la revendication 17, dans lequel R₅ est un groupe méthyle ou éthyle.

19. Composé selon la revendication 1, choisi dans le groupe constitué des suivants:
5-chloro-2-(3,5-di-t-butyl-4-hydroxy-benzyl)-7-(2-(2-pyridyl)-éthynyl)benzoxazole,
2-((3,5-di-t-butyl-4-hydroxy)benzyl)-7-(2-(2-pyridyl)-éthynyl)benzoxazole,
2-((3,5-di-isopropyl-4-hydroxy)benzyl)-7-(2-(2-pyridyl)-éthynyl)benzoxazole.

20. Utilisation d'un composé selon l'une quelconque des revendications précédentes dans la préparation d'un médicament destiné à une méthode pour réaliser une inhibition de PDE IV sélective chez un patient nécessitant cela, qui comprend l'administration d'une quantité efficace du composé.

21. Utilisation selon la revendication 20, dans laquelle ledit composé est choisi dans le groupe constitué des suivants:
5-chloro-2-(3,5-di-t-butyl-4-hydroxy-benzyl)-7-(2-(2-pyridyl)-éthynyl)benzoxazole,
2-((3,5-di-t-butyl-4-hydroxy)benzyl)-7-(2-(2-pyridyl)-éthynyl)benzoxazole, et
2-((3,5-di-isopropyl-4-hydroxy)benzyl)-7-(2-(2-pyridyl)-éthynyl)benzoxazole.

22. Composition pharmaceutique comprenant un composé ayant la structure chimique présentée dans la revendication 1.

23. Composition pharmaceutique selon la revendication 22, dans laquelle ledit composé est choisi dans le groupe constitué des suivants:
5-chloro-2-(3,5-di-t-butyl-4-hydroxy-benzyl)-7-(2-(2-pyridyl)-éthynyl)benzoxazole,
2-((3,5-di-t-butyl-4-hydroxy)benzyl)-7-(2-(2-pyridyl)-éthynyl)benzoxazole, et
2-((3,5-di-isopropyl-4-hydroxy)benzyl)-7-(2-(2-pyridyl)-éthynyl)benzoxazole.

24. Utilisation d'un composé selon l'une quelconque des revendications 1 à 19 dans la préparation d'un médicament destiné à une méthode de traitement d'un mammifère souffrant d'un état pathologique choisi dans le groupe constitué de l'asthme, des allergies, de l'inflammation, de la dépression, de la démence, des maladies atopiques, de la rhinite et des états pathologiques associés à des taux physiologiques anormalement élevés d'un membre du groupe constitué des cytokines, des cytokines inflammatoires et des chémokines, comprenant l'administration d'une quantité efficace du composé.

25. Utilisation selon la revendication 24, dans laquelle ladite composition est choisie dans le groupe constitué des composés suivants:
5-chloro-2-(3,5-di-t-butyl-4-hydroxy-benzyl)-7-(2-(2-pyridyl)-éthynyl)benzoxazole,
2-((3,5-di-t-butyl-4-hydroxy)benzyl)-7-(2-(2-pyridyl)-éthynyl)benzoxazole, et
2-((3,5-di-isopropyl-4-hydroxy)benzyl)-7-(2-(2-pyridyl)-éthynyl)benzoxazole.

26. Composé selon la revendication 1 ayant une CI₅₀ de PDE IV inférieure à 10 µM.
